# EUROPEAN PATENT APPLICATION

(11) **EP 1 967 505 A1**
(43) Date of publication of application: **10.09.2008**
(21) Application number: 08002869.9
(22) Date of filing: 15.02.2008
(51) Int. Cl.: C07C 37/50, C07C 39/07, C07C 39/08

(54) **Process for the preparation of substituted phenols**

(30) Priority: 08.03.2007 JP 2007059228
(71) Applicant: Daicel Chemical Industries, Ltd., Kita-ku, Osaka-shi Osaka 530-0001 (JP)
(72) Inventor: Ishii, Yasutaka, Takatsuki-shi Osaka 569-1112 (JP); Iwahama, Takahiro, Himeji-shi Hyogo 671-1283 (JP)
(74) Representative: Grünecker, Kinkeldey, Stockmair & Schwanhäusser Anwaltssozietät

(57) **Abstract**

A substituted phenolic compound is prepared by oxidizing a substituted diarylethane compound with oxygen in the presence of a nitrogen-containing cyclic compound, and treating the oxidized product with an acid. The nitrogen-containing cyclic compound includes, as a constituent of its ring, a skeleton represented by following Formula (I): 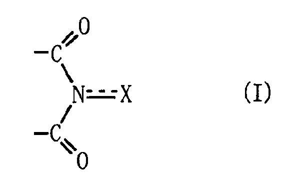 wherein X is oxygen atom or an -OR group, where R is hydrogen atom or a hydroxyl-protecting group. The substituted diarylethane compound is represented by following Formula (1): 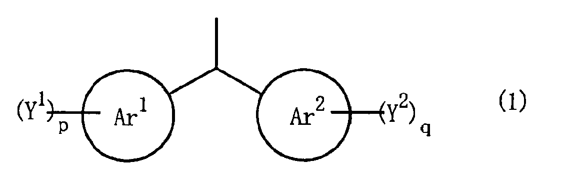 wherein each of Ring Ar¹ and Ring Ar² is independently a monocyclic or polycyclic aromatic carbocyclic ring; Y¹ is an electron-donating group; Y² is an electron-withdrawing group; "p" is an integer of 1 or more; and "q" is an integer of 0 or more. The substituted phenolic compound is represented by following Formula (2): 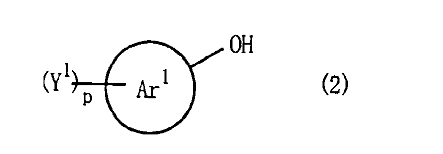 wherein Ring Ar¹, Y¹, and "p" are as defined above.

## Description

### BACKGROUND OF THE INVENTION

### 1. Field of the Invention

The present invention relates to processes for the preparation of substituted phenolic compounds that are useful typically as materials for phenolic resins, synthetic dyestuffs, plasticizers, medicaments, and agricultural chemicals.

### 2. Description of the Related Art

Phenol has been industrially produced by oxidizing cumene. In addition, Adv. Synth. Catal., 2001, 343, 809 reports a process for the preparation of phenol through oxidation of cumene catalyzed by N-hydroxyphthalimide. There are, however, few processes for efficiently producing substituted phenolic compounds each having one or more substituents such as alkyl groups and alkoxy groups on their benzene ring.

### SUMMARY OF THE INVENTION

Accordingly, it is desirable to provide a process for efficiently preparing a substituted phenolic compound having one or more substituents such as an alkyl group and an alkoxy group on its aromatic carbocyclic ring.

After intensive investigations, the present inventors have found that a substituted phenolic compound is selectively produced by oxidizing a substituted diarylethane compound by the catalysis of an imide catalyst, in which the substituted diarylethane compound is easily available through a reaction between a substituted aromatic hydrocarbon and an aromatic vinyl compound, and the resulting substituted phenolic compound corresponds to the substituted aromatic hydrocarbon. The present invention has been made based on these findings.

Specifically, according to an embodiment of the present invention, there is provided a process for the preparation of a substituted phenolic compound. This process includes the steps of oxidizing a substituted diarylethane compound with oxygen in the presence of a nitrogen-containing cyclic compound to give an oxidized product, and treating the oxidized product with an acid. The nitrogen-containing cyclic compound contains, as a constituent of its ring, a skeleton represented by following Formula (I): 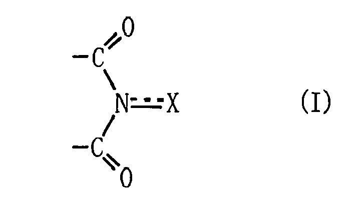 wherein X represents oxygen atom or an -OR group, where R represents hydrogen atom or a hydroxyl-protecting group. The substituted diarylethane compound is represented by following Formula (1): 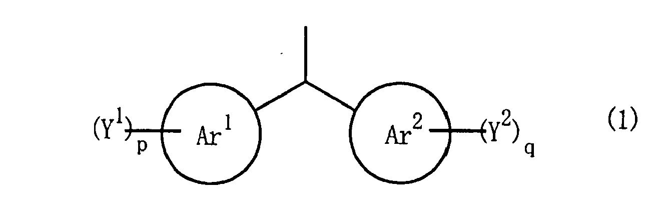 wherein Ring Ar¹ and Ring Ar² each independently represent a monocyclic or polycyclic aromatic carbocyclic ring; Y¹ represents an electron-donating group bound to Ring Ar¹; Y² represents an electron-withdrawing group bound to Ring Ar²; "p" denotes an integer of 1 or more; and "q" denotes an integer of 0 or more, in which, when "p" is an integer of 2 or more and there are two or more Y¹s, the two or more Y¹s may be the same as or different from one another and they may be combined to form a ring with carbon atom on Ring Ar¹, and when "q" is an integer of 2 or more and there are two or more Y²s, the two or more Y²s may be the same as or different from one another and they may be combined to form a ring with carbon atom on Ring Ar². The substituted phenolic compound is represented by following Formula (2): 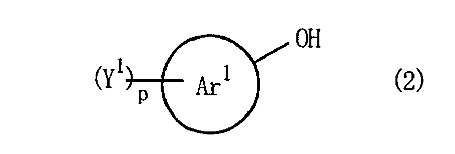 wherein Ring Ar¹, Y¹, and "p" are as defined above.

Such nitrogen-containing cyclic compounds include for use herein, for example, a compound represented by following Formula (3): 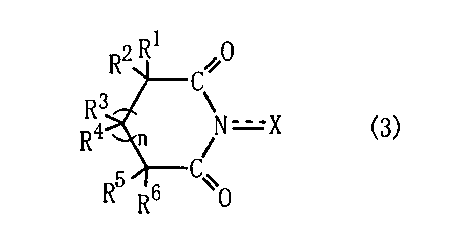 wherein "n" is 0 or 1; X represents oxygen atom or an -OR group, where R represents hydrogen atom or a hydroxyl-protecting group; and R¹, R², R³, R⁴, R⁵, and R⁶ each independently represent one selected from the group consisting of hydrogen atom, a halogen atom, an alkyl group, an aryl group, a cycloalkyl group, hydroxyl group, an alkoxy group, carboxyl group, a substituted oxycarbonyl group, an acyl group, and an acyloxy group, in which at least two of R¹, R², R³, R⁴, R⁵, and R⁶ may be combined to form a double bond, or an aromatic or non-aromatic ring together with carbon atom or carbon-carbon bond constituting the cyclic imide skeleton, and one or more N-substituted cyclic imido groups may be further formed on at least one of R¹, R², R³, R⁴, R⁵, and R⁶, and/or on at least one of the double bond and the aromatic or non-aromatic ring formed by at least two of R¹, R², R³, R⁴, R⁵, and R⁶, where the N-substituted cyclic imido groups are each represented by following Formula (a): 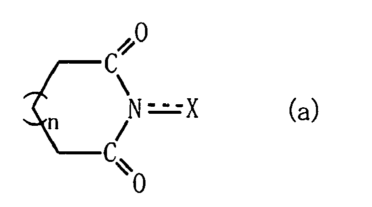 wherein "n" and X are as defined above.

The electron-donating group as Y¹ may be a substituent selected from an alkyl group, a cycloalkyl group, hydroxyl group, mercapto group, a substituted oxy group, a substituted thio group, amino group, and a mono- or di-substituted amino group.

According to an embodiment of the present invention, a variety of substituted phenolic compounds can be efficiently obtained from substituted diarylethane compounds in good yields, in which the substituted diarylethane compounds are easily available through reactions between substituted aromatic hydrocarbons and aromatic vinyl compounds.

### DESCRIPTION OF THE PREFERRED EMBODIMENTS

### [Substituted Diarylethane Compound]

A substituted diarylethane compound used as a starting material herein is represented by Formula (1). In Formula (1), Ring Ar¹ and Ring Ar² are each a monocyclic or polycyclic aromatic carbocyclic ring; Y¹ is an electron-donating group bound to Ring Ar¹; Y² is an electron-withdrawing group bound to Ring Ar²; "p" is an integer of 1 or more; and "q" is an integer of 0 or more. When "p" is an integer of 2 or more and there are two or more Y¹s, the two or more Y¹s may be the same as or different from one another, and they may be combined to form a ring with carbon atom on Ring Ar¹ (carbon atom constituting Ring Ar¹). When "q" is an integer of 2 or more and there are two or more Y²s, the two or more Y²s may be the same as or different from one another, and they may be combined to form a ring with carbon atom on Ring Ar² (carbon atom constituting Ring Ar²).

Examples of the monocyclic or polycyclic aromatic carbocyclic rings as Ring Ar¹ and Ring Ar² include aromatic carbocyclic rings each having one to ten rings, such as benzene ring, naphthalene ring, phenanthrene ring, anthracene ring, and naphthacene ring. Among them, aromatic carbocyclic rings having one to five rings are preferred, of which benzene ring and naphthalene ring are more preferred.

Examples of the electron-donating groups as Y¹ include alkyl groups, cycloalkyl groups, hydroxyl group, mercapto group, substituted oxy groups, substituted thio groups, amino group, and mono- or di-substituted amino groups. Examples of the alkyl groups include straight- or branched-chain alkyl groups having about one to about twenty carbon atoms, such as methyl, ethyl, propyl, isopropyl, butyl, isobutyl, s-butyl, t-butyl, hexyl, decyl, dodecyl, tetradecyl, and hexadecyl groups, of which those having about one to about twelve carbon atoms are preferred, and those having about one to about six carbon atoms are more preferred. Examples of the cycloalkyl groups include cycloalkyl groups having about three to about twenty members, such as cyclopropyl, cyclobutyl, cyclopentyl, cyclohexyl, cyclooctyl, cyclodecyl, and cyclododecyl groups, of which those having three to fifteen members are preferred, and those having five or six members are more preferred.

Examples of the substituted oxy groups include alkoxy groups having about one to about twenty carbon atoms, such as methoxy, ethoxy, isopropoxy, butoxy, t-butoxy, hexyloxy, octyloxy, decyloxy, dodecyloxy, tetradecyloxy, and octadecyloxy groups, of which those having about one to about twelve carbon atoms are preferred, and those having about one to about six carbon atoms are more preferred; acyloxy groups having about one to about twenty carbon atoms, such as formyloxy, acetyloxy, propionyloxy, and benzoyloxy groups, of which those having about one to about twelve carbon atoms are preferred, and those having about one to about six carbon atoms are more preferred; sulfonyloxy groups such as benzenesulfonyloxy and p-toluenesulfonyloxy groups; and substituted oxycarbonyloxy groups, including alkoxycarbonyloxy groups and aryloxycarbonyloxy groups, having about two to about twenty-one carbon atoms, such as methoxycarbonyloxy, ethoxycarbonyloxy, and phenoxycarbonyloxy groups, of which those having about two to about thirteen carbon atoms are preferred, and those having about two to about seven carbon atoms are more preferred. Examples of the substituted thio groups include alkylthio groups having about one to about twenty carbon atoms, such as methylthio, ethylthio, butylthio, and decylthio groups, of which those having about one to about twelve carbon atoms are preferred, and those having about one to about six carbon atoms are more preferred. Examples of the mono- or di-substituted amino groups include mono- or di-alkylamino groups such as methylamino, dimethylamino, ethylamino, and diethylamino groups; cyclic amino groups such as 1-pyrrolidinyl, piperidino, and morpholino groups; acylamino groups such as acetylamino, propionylamino, and benzoylamino groups; and sulfonylamino groups such as benzenesulfonylamino and p-toluenesulfonylamino groups.

When there are two or more Y¹s, the two or more Y¹ may be combined to form a ring with carbon atom on Ring Ar¹. Examples of the ring herein include cycloalkane rings having three to eight members, such as cyclopropane ring, cyclopentane ring, and cyclohexane ring; as well as oxetane ring, oxolane ring, oxane ring, dioxolane ring, and dioxane ring. Each of these rings may have one or more substituents. Examples of the substituents include alkyl groups such as methyl and ethyl groups, of which alkyl groups having about one to about six carbon atoms are preferred; alkoxy groups such as methoxy and ethoxy group; and halogen atoms such as fluorine, chlorine, and bromine atoms.

The position(s) of the substituent(s) Y¹ is not particularly limited, but is preferably the ortho position or para position, or a corresponding position when Ring Ar¹ is a polycyclic ring, to the 1-arylethyl group whose aryl moiety, i.e., Ring Ar² may have one or more substituents Y²s. This is because a substituted diarylethane compound of this type as a starting material can be easily prepared and shows satisfactory selectivity in reaction. When there are two or more Y¹s, at least one of them is preferably at the ortho position or para position to the 1-arylethyl group whose aryl moiety, i.e., Ring Ar² may have one or more substituents Y²s. The number "p" of Y¹ is an integer of 1 or more, and is preferably an integer of 1 to 3, and more preferably 1 or 2.

Examples of electron-withdrawing groups as Y²(s) include halogen atoms, haloalkyl groups, aryl groups, carboxyl group, substituted oxycarbonyl groups, acyl groups, cyano group, nitro group, sulfo group, and substituted oxysulfonyl groups. The halogen atoms include fluorine atom, chlorine atom, bromine atom, and iodine atom. The haloalkyl groups include haloalkyl groups having about one to about twenty carbon atoms, such as fluoromethyl, difluoromethyl, trifluoromethyl, chloromethyl, dichloromethyl, trichloromethyl, and bromomethyl group, of which those having about one to about twelve carbon atoms are preferred, and those having about one to about six carbon atoms are more preferred.

The aryl groups include phenyl, tolyl, xylyl, and naphthyl groups. The substituted oxycarbonyl groups include alkoxy-carbonyl groups whose alkoxy moiety has about one to about twenty carbon atoms, such as methoxycarbonyl, ethoxycarbonyl, isopropoxycarbonyl, butoxycarbonyl, t-butoxycarbonyl, and hexyloxycarbonyl groups, of which alkoxy-carbonyl groups whose alkoxy moiety has about one to about twelve carbon atom are preferred, and alkoxy-carbonyl groups whose alkoxy moiety has about one to about six carbon atoms are more preferred; aryloxycarbonyl groups such as phenyloxycarbonyl and naphthyloxycarbonyl groups, of which aryloxy-carbonyl groups whose aryloxy moiety has about six to about twenty carbon atoms are preferred; and aralkyloxycarbonyl groups such as benzyloxycarbonyl group, of which aralkyloxy-carbonyl groups whose araylkyloxy moiety has about seven to about twenty-one carbon atoms are preferred. The acyl groups include aliphatic acyl groups having about one to about twenty carbon atoms, such as formyl, acetyl, propionyl, butyryl, isobutyryl, valeryl, pivaloyl, and hexanoyl groups, of which those having about one to about twelve carbon atoms are preferred, and those having about one to about six carbon atoms are more preferred; and aromatic acyl groups such as benzoyl and naphthoyl groups. The substituted oxysulfonyl groups include substituted oxysulfonyl groups having about one to about twenty carbon atoms, such as methoxysulfonyl group and ethoxysulfonyl group, of which those having about one to about twelve carbon atoms are preferred, and those having about one to about six carbon atoms are more preferred.

When there are two or more Y²s, the two or more Y²s may be combined to form a ring with carbon atom on Ring Ar². Examples of the ring herein include cyclic acid anhydride skeletons.

The position(s) of Y²(s) are not particularly limited. The number "q" of Y² is an integer of 0 or more. It is preferably an integer of 0 to 3, and more preferably an integer of 0 to 2.

Representative examples of the substituted diarylethane compound of Formula (1) include 1-methyl-4-(1-phenylethyl)benzene, 1,2-dimethyl-4-(1-phenylethyl)benzene, 1,3-dimethyl-4-(1-phenylethyl)benzene, 1-t-butyl-4-(1-phenylethyl)benzene, 4-[1-(4-chlorophenyl)ethyl]-1,2-dimethylbenzene, 4-(1-phenylethyl)anisole, 1,2-dimethoxy-4-(1-phenylethyl)benzene, 1,4-dimethoxy-3-(1-phenylethyl)benzene, 1-methylthio-4-(1-phenylethyl)benzene, 1-cyclohexyl-4-(1-phenylethyl)benzene, 4-(1-phenylethyl)phenol, 4-(1-phenylethyl)thiophenol, 1-methylthio-4-(1-phenylethyl)benzene, 4-(1-phenylethyl)aniline, N-methyl-4-(1-phenylethyl)aniline, N,N-dimethyl-4-(1-phenylethyl)aniline, 2-methyl-6-(1-phenylethyl)naphthalene, and 2-methoxy-6-(1-phenylethyl)naphthalene.

A substituted diarylethane compound of Formula (1) can be prepared, for example, through a reaction between a substituted aromatic hydrocarbon (e.g., a substituted benzene or a substituted naphthalene) and an aromatic vinyl compound (e.g., a styrenic compound or a vinylnaphthalene compound), in which the substituted aromatic hydrocarbon is represented by following Formula (4): 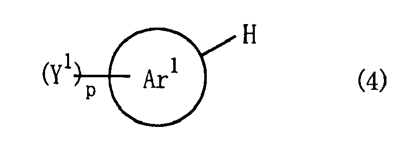 wherein Ring Ar¹, Y¹, and "p" are as defined above, and the aromatic vinyl compound is represented by following Formula (5) : 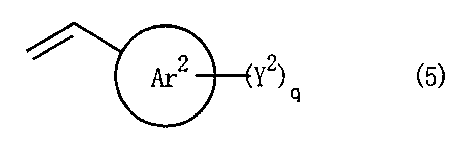 wherein Ring Ar², Y², and "q" are as defined above.

This reaction is conducted in the presence of, or in the absence of, a solvent. The solvent is not particularly limited, as long as it does not adversely affect the reaction. The reaction is generally conducted in the presence of an acid catalyst. Examples of the acid catalyst include Lewis acids such as ferric chloride, ferric bromide, aluminum chloride, titanium chloride, zirconium chloride, zinc chloride, and tin chloride; and Broensted acids such as sulfuric acid.

The ratio in amount of the substituted aromatic hydrocarbon of Formula (4) to the aromatic vinyl compound of Formula (5) is not particularly limited and can be appropriately set according typically to cost. The substituted aromatic hydrocarbon of Formula (4) is generally used in excess. The reaction is conducted at a temperature of, for example, about 30°C to about 150°C, preferably about 40°C to about 120°C, and more preferably about 50°C to about 100°C.

After the completion of reaction, the formed substituted diarylethane compound can be obtained, for example, by removing the acid catalyst typically through washing with water and carrying out distillation.

### [Nitrogen-Containing Cyclic Compound]

A preparation process according to an embodiment of the present invention uses, as a catalyst, a nitrogen-containing cyclic compound containing a skeleton of Formula (I) as a constituent of its ring. In Formula (I), the bond between nitrogen atom and X is single bond or double bond. X is oxygen atom or an -OR group, where R is hydrogen atom or a hydroxyl-protecting group. The nitrogen-containing cyclic compound may have two or more skeletons of Formula (I) per molecule. The nitrogen-containing cyclic compound, when X is an -OR group and R is a hydroxyl-protecting group, may have two or more moieties bound through R, in which the two or more moieties each correspond to the skeleton of Formula (I) where X is an -OR group other than R.

The hydroxyl-protecting group represented by R in Formula (I) can be a hydroxyl-protecting group commonly used in organic synthesis. Such protecting groups include alkyl groups (e.g., alkyl groups having about one to about four carbon atoms, such as methyl and t-butyl groups), alkenyl groups (e.g., allyl group), cycloalkyl groups (e.g., cyclohexyl group), aryl groups (e.g., 2,4-dinitrophenyl group), and aralkyl groups (e.g., benzyl, 2,6-dichlorobenzyl, 3-bromobenzyl, 2-nitrobenzyl, and triphenylmethyl group); groups capable of forming acetal or hemiacetal group with hydroxyl group, including substituted methyl groups (e.g., methoxymethyl, methylthiomethyl, benzyloxymethyl, t-butoxymethyl, 2-methoxyethoxymethyl, 2,2,2-trichloroethoxymethyl, bis(2-chloroethoxy)methyl, and 2-(trimethylsilyl)ethoxymethyl groups), substituted ethyl groups (e.g., 1-ethoxyethyl, 1-methyl-l-methoxyethyl, 1-isopropoxyethyl, 2,2,2-trichloroethyl, and 2-methoxyethyl groups), tetrahydropyranyl group, tetrahydrofuranyl group, and 1-hydroxyalkyl groups (e.g., 1-hydroxyethyl, 1-hydroxyhexyl, 1-hydroxydecyl, 1-hydroxyhexadecyl, and 1-hydroxy-1-phenylmethyl groups); acyl groups (e.g., aliphatic saturated or unsaturated acyl groups including aliphatic acyl groups having about one to about twenty carbon atoms, such as formyl, acetyl, propionyl, butyryl, isobutyryl, valeryl, pivaloyl, hexanoyl, heptanoyl, octanoyl, nonanoyl, decanoyl, lauroyl, myristoyl, palmitoyl, and stearoyl groups; acetoacetyl group; alicyclic acyl groups including cycloalkanecarbonyl groups, such as cyclopentanecarbonyl and cyclohexanecarbonyl groups; and aromatic acyl groups such as benzoyl and naphthoyl groups; sulfonyl groups (e.g., methanesulfonyl, ethanesulfonyl, trifluoromethanesulfonyl, benzenesulfonyl, p-toluenesulfonyl, and naphthalenesulfonyl groups), alkoxycarbonyl groups (e.g., alkoxy-carbonyl groups whose alkoxy moiety has about one to about four carbon atoms, such as methoxycarbonyl, ethoxycarbonyl, and t-butoxycarbonyl groups), aralkyloxycarbonyl groups (e.g., benzyloxycarbonyl group and p-methoxybenzyloxycarbonyl group), substituted or unsubstituted carbamoyl groups (e.g., carbamoyl, methylcarbamoyl, and phenylcarbamoyl groups), groups corresponding to moieties of inorganic acids (e.g., sulfuric acid, nitric acid, phosphoric acid, and boric acid) other than hydroxyl group (OH group), dialkylphosphinothioyl groups (e.g., dimethylphosphinothioyl group), diarylphosphinothioyl groups (e.g., diphenylphosphinothioyl group), and substituted silyl groups (e.g., trimethylsilyl, t-butyldimethylsilyl, tribenzylsilyl, and triphenylsilyl groups).

When X is an -OR group and two or more moieties corresponding to the skeleton of Formula (I) other than R are bound through R, examples of the group R include polycarboxylic acid acyl groups, such as oxalyl, malonyl, succinyl, glutaryl, phthaloyl, isophthaloyl, and terephthaloyl groups; carbonyl group; and polyvalent hydrocarbon groups such as methylene, ethylidene, isopropylidene, cyclopentylidene, cyclohexylidene, and benzylidene groups, of which groups capable of forming acetal with two hydroxyl groups are preferred.

Preferred examples of R include hydrogen atom; groups capable of forming acetal or hemiacetal group with hydroxyl group; hydrolyzable protecting groups that can leave as a result of hydrolysis, such as groups corresponding to moieties of acids (e.g., carboxylic acids, sulfonic acids, carbonic acid, carbamic acid, sulfuric acid, phosphoric acid, and boric acid) other than hydroxyl group, such as acyl groups, sulfonyl groups, alkoxycarbonyl groups, and carbamoyl groups. R is particularly preferably hydrogen atom.

Such nitrogen-containing cyclic compounds each having a skeleton of Formula (I) as a constituent of its ring include cyclic imide compounds each having a cyclic imide skeleton of Formula (3). In Formula (3), "n" denotes 0 or 1. X is oxygen atom or an -OR group, where R is hydrogen atom or a hydroxyl-protecting group. Each of R¹, R², R³, R⁴, R⁵ and R⁶ independently represents hydrogen atom, a halogen atom, an alkyl group, an aryl group, a cycloalkyl group, hydroxyl group, an alkoxy group, carboxyl group, a substituted oxycarbonyl group, an acyl group, or an acyloxy group. At least two of R¹, R², R³, R⁴, R⁵ and R⁶ may be combined to form a double bond, an aromatic or non-aromatic ring together with carbon atom or carbon-carbon bond constituting the cyclic imide skeleton. On the substituents R¹, R², R³, R⁴, R⁵, R⁶, and/or on the double bond, or aromatic or non-aromatic ring formed by at least two of R¹, R², R³, R⁴, R⁵ and R⁶, there may be further formed one or more cyclic imido groups of Formula (a) where "n" and X are as defined above.

In cyclic imide compounds of Formula (3), the halogen atoms as the substituents R¹, R², R³, R⁴, R⁵ and R⁶ include iodine, bromine, chlorine, and fluorine atoms. The alkyl groups include straight- or branched-chain alkyl groups having about one to about thirty carbon atoms, such as methyl, ethyl, propyl, isopropyl, butyl, hexyl, decyl, and dodecyl groups, of which those having about one to about twenty carbon atoms are preferred.

The aryl groups include phenyl, tolyl, xylyl, and naphthyl groups. The cycloalkyl groups include cyclopentyl and cyclohexyl groups. The alkoxy groups include alkoxy groups having about one to about thirty carbon atoms, such as methoxy, ethoxy, isopropoxy, butoxy, t-butoxy, hexyloxy, decyloxy, and dodecyloxy groups, of which those having about one to about twenty carbon atoms are preferred.

The substituted oxycarbonyl groups include alkoxy-carbonyl groups whose alkoxy moiety has about one to about thirty carbon atoms, such as methoxycarbonyl, ethoxycarbonyl, isopropoxycarbonyl, butoxycarbonyl, t-butoxycarbonyl, hexyloxycarbonyl, and decyloxycarbonyl groups, of which alkoxy-carbonyl groups whose alkoxy moiety has about one to about twenty carbon atoms are preferred; cycloalkyloxycarbonyl groups such as cyclopentyloxycarbonyl and cyclohexyloxycarbonyl groups, of which those having about three to about twenty members are preferred; aryloxycarbonyl groups such as phenyloxycarbonyl group, of which aryloxy-carbonyl groups whose aryloxy moiety has about six to about twenty carbon atoms are preferred; and aralkyloxycarbonyl groups such as benzyloxycarbonyl group, of which aralkyloxy-carbonyl groups whose aralkyloxy moiety has about seven to about twenty-one carbon atoms are preferred.

The acyl groups include aliphatic saturated or unsaturated acyl groups including aliphatic acyl groups having about one to about thirty carbon atoms, such as formyl, acetyl, propionyl, butyryl, isobutyryl, valeryl, pivaloyl, hexanoyl, decanoyl, and lauroyl groups, of which those having about one to about twenty carbon atoms are preferred; acetoacetyl group; alicyclic acyl groups including cycloalkanecarbonyl groups such as cyclopentanecarbonyl and cyclohexanecarbonyl groups; and aromatic acyl groups such as benzoyl group.

Examples of the acyloxy groups include saturated or unsaturated aliphatic acyloxy groups including aliphatic acyloxy groups having about one to about thirty carbon atoms, such as formyloxy, acetyloxy, propionyloxy, butyryloxy, isobutyryloxy, valeryloxy, pivaloyloxy, decanoyloxy, and lauroyloxy groups, of which those having about one to about twenty carbon atoms are preferred; acetoacetyloxy group; alicyclic acyloxy groups including cycloalkanecarbonyloxy groups such as cyclopentanecarbonyloxy and cyclohexanecarbonyloxy groups; and aromatic acyloxy groups such as benzoyloxy group.

The substituents R¹, R², R³, R⁴, R⁵, and R⁶ may be the same as or different from one another. At least two of R¹, R², R³, R⁴, R⁵, and R⁶ in Formula (3) may be combined to form a double bond, or an aromatic or non-aromatic ring together with carbon atom or carbon-carbon bond constituting the cyclic imide skeleton. The aromatic or non-aromatic ring is preferably a ring having about five to about twelve members and is more preferably a ring having about six to about ten members. The ring may be a heterocyclic ring or fused heterocyclic ring but is often a hydrocarbon ring. Examples of such rings include non-aromatic alicyclic rings including substituted or unsubstituted cycloalkane rings such as cyclohexane ring, and substituted or unsubstituted cycloalkene rings such as cyclohexene ring; non-aromatic bridged rings including substituted or unsubstituted bridged hydrocarbon rings such as 5-norbornene ring; substituted or unsubstituted aromatic rings (including fused rings) such as benzene ring and naphthalene ring. The ring is often composed of an aromatic ring. The ring may be substituted by one or more substituents. Examples of the substituents are alkyl groups, haloalkyl groups, hydroxyl group, alkoxy groups, carboxyl group, substituted oxycarbonyl groups, acyl groups, acyloxy groups, nitro group, cyano group, amino group, and halogen atoms.

One or more N-substituted cyclic imido groups of Formula (a) may be further formed on at least one of R¹, R², R³, R⁴, R⁵, and R⁶ and/or on the double bond, or aromatic or non-aromatic ring formed by at least two of R¹, R², R³, R⁴, R⁵, and R⁶. For example, when at least one of R¹, R², R³, R⁴, R⁵, and R⁶ is an alkyl group containing two or more carbon atoms, the N-substituted cyclic imido group may be formed as containing adjacent two carbon atoms constituting the alkyl group. Likewise, when at least two of R¹, R², R³, R⁴, R⁵, and R⁶ are combined to form a double bond with carbon-carbon bond constituting the cyclic imide skeleton, the N-substituted cyclic imido group may be formed as containing the double bond. When at least two of R¹, R², R³, R⁴, R⁵, and R⁶ are combined to form an aromatic or non-aromatic ring with carbon atom or carbon-carbon bond constituting the cyclic imide skeleton, the N-substituted cyclic imido group may be formed as containing adjacent two carbon atoms constituting the ring.

Preferred cyclic imide compounds include compounds of following formulae: 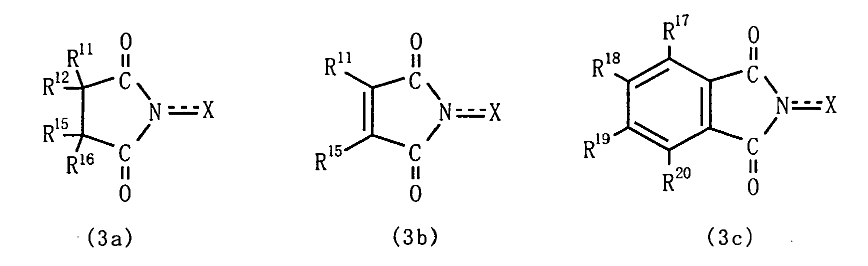 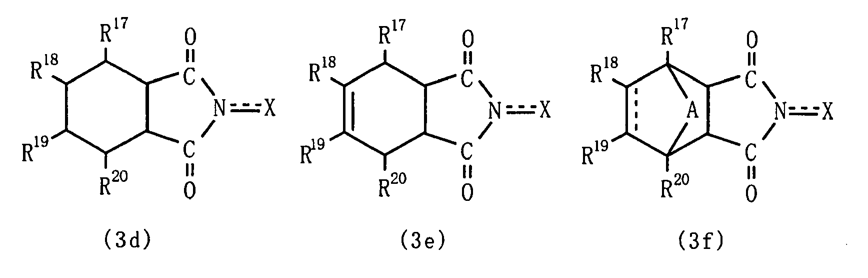 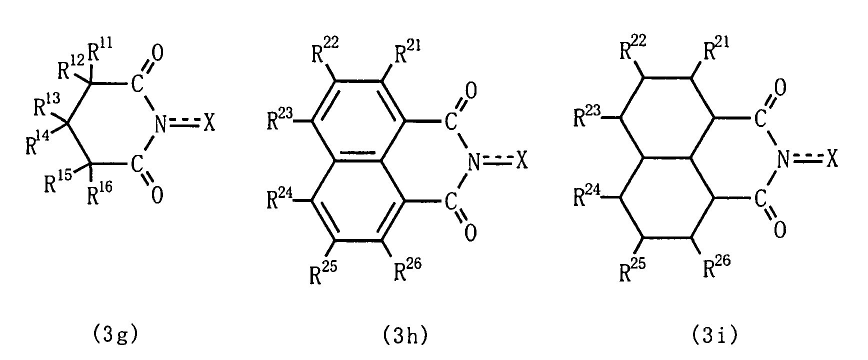 wherein R¹¹, R¹², R¹³, R¹⁴, R¹⁵, and R¹⁶ are each independently one of hydrogen atom, a halogen atom, an alkyl group, an aryl group, a cycloalkyl group, hydroxyl group, an alkoxy group, carboxyl group, a substituted oxycarbonyl group, an acyl group, and an acyloxy group; R¹⁷, R¹⁸, R¹⁹, R²⁰, R²¹, R²², R²³, R²⁴, R²⁵, and R²⁶ are each independently one of hydrogen atom, an alkyl group, a haloalkyl group, hydroxyl group, an alkoxy group, carboxyl group, a substituted oxycarbonyl group, an acyl group, an acyloxy group, nitro group, cyano group, amino group, and a halogen atom, in which adjacent two of R¹⁷ to R²⁶ may be combined to form a five- or six-membered N-substituted cyclic imide skeleton shown in one of Formulae (3c), (3d), (3e), (3f), (3h), and (3i); "A" in Formula (3f) is methylene group or oxygen atom; and X is as defined above.

Examples of the halogen atoms, alkyl groups, aryl groups, cycloalkyl groups, hydroxyl group, alkoxy groups, carboxyl group, substituted oxycarbonyl groups, acyl groups, and acyloxy groups as the substituents R¹¹ to R¹⁶ are as with the corresponding groups in connection with the substituents R¹ to R⁶.

As the substituents R¹⁷ to R²⁶, examples of the alkyl groups are as above, of which alkyl groups having about one to about six carbon atoms are preferred. The haloalkyl groups include haloalkyl groups having about one to about four carbon atoms, such as trifluoromethyl group. Examples of the alkoxy groups are as above, of which lower alkoxy groups having about one to about four carbon atoms are preferred. Examples of the substituted oxycarbonyl groups are as above, such as alkoxycarbonyl groups, cycloalkyloxycarbonyl groups, aryloxycarbonyl groups, and aralkyloxycarbonyl groups. Examples of the acyl groups are as above, such as saturated or unsaturated aliphatic acyl groups, acetoacetyl group, alicyclic acyl groups, and aromatic acyl groups. Examples of the acyloxy groups are as above, such as saturated or unsaturated aliphatic acyloxy groups, acetoacetyloxy group, alicyclic acyloxy groups, and aromatic acyloxy groups. The halogen atoms include fluorine, chlorine, and bromine atoms. The substituents R¹⁷ to R²⁶ are each often one of hydrogen atom, a lower alkyl group having about one to about four carbon atoms, carboxyl group, a substituted oxycarbonyl group, nitro group, and a halogen atom.

Of preferred imide compounds, representative examples of compounds having a five-membered N-substituted cyclic imide skeleton include compounds of Formula (3) where X is an -OR group and R is hydrogen atom, such as N-hydroxysuccinimide, N-hydroxy-α-methylsuccinimide, N-hydroxy-α,α-dimethylsuccinimide, N-hydroxy-α,β-dimethylsuccinimide, N-hydroxy-α,α,β,β-tetramethylsuccinimide, N-hydroxymaleimide, N-hydroxyhexahydrophthalimide, N,N'-dihydroxycyclohexanetetracarboxylic diimide, N-hydroxyphthalimide, N-hydroxytetrabromophthalimide, N-hydroxytetrachlorophthalimide, N-hydroxychlorendimide, N-hydroxyhimimide, N-hydroxytrimellitimide, N,N'-dihydroxypyromellitic diimide, N,N'-dihydroxynaphthalenetetracarboxylic diimide, α,β-diacetoxy-N-hydroxysuccinimide, N-hydroxy-α,β-bis(propionyloxy)succinimide, N-hydroxy-α,β-bis(valeryloxy)succinimide, N-hydroxy-α,β-bis(lauroyloxy)succinimide, α,β-bis(benzoyloxy)-N-hydroxysuccinimide, N-hydroxy-4-methoxycarbonylphthalimide, 4-chloro-N-hydroxyphthalimide, 4-ethoxycarbonyl-N-hydroxyphthalimide, N-hydroxy-4-pentyloxycarbonylphthalimide, 4-dodecyloxy-N-hydroxycarbonylphthalimide, N-hydroxy-4-phenoxycarbonylphthalimide, N-hydroxy-4,5-bis(methoxycarbonyl)phthalimide, 4,5-bis(ethoxycarbonyl)-N-hydroxyphthalimide, N-hydroxy-4,5-bis(pentyloxycarbonyl)phthalimide, 4,5-bis(dodecyloxycarbonyl)-N-hydroxyphthalimide, and N-hydroxy-4,5-bis(phenoxycarbonyl)phthalimide; compounds corresponding to these compounds, except with R being an acyl group such as acetyl group, propionyl group, or benzoyl group; compounds of Formula (3) where X is an -OR group and R is a group capable of forming acetal or hemiacetal bond with hydroxyl group, such as N-methoxymethyloxyphthalimide, N-(2-methoxyethoxymethyloxy)phthalimide, and N-tetrahydropyranyloxyphthalimide; compounds of Formula (3) where X is an -OR group and R is sulfonyl group, such as N-methanesulfonyloxyphthalimide and N-(p-toluenesulfonyloxy)phthalimide; and compounds of Formula (3) where X is an -OR group and R is a group corresponding to a moiety of an inorganic acid other than hydroxyl group, such as sulfuric ester, nitric ester, phosphoric ester, and boric ester of N-hydroxyphthalimide.

Of preferred imide compounds, representative examples of compounds each having a six-membered N-substituted cyclic imide skeleton include compounds of Formula (3) where X is an -OR group and R is hydrogen atom, such as N-hydroxyglutarimide, N-hydroxy-α,α-dimethylglutarimide, N-hydroxy-β,β-dimethylglutarimide, N-hydroxy-1,8-decalindicarboximide, N,N'-dihydroxy-1,8;4,5-decalintetracarboxylic diimide, N-hydroxy-1,8-naphthalenedicarboximide (N-hydroxynaphthalimide), and N,N'-dihydroxy-1,8;4,5-naphthalenetetracarboxylic diimide; compounds corresponding to these compounds, except with R being an acyl group such as acetyl group, propionyl group, or benzoyl group; compounds of Formula (3) where X is an -OR group and R is a group capable of forming acetal or hemiacetal bond with hydroxyl group, such as N-methoxymethyloxy-1,8-naphthalenedicarboximide, N,N'-bis(methoxymethyloxy)-1,8;4,5-naphthalenetetracarboxylic diimide; compounds of Formula (3) where X is an -OR group and R is sulfonyl group, such as N-methanesulfonyloxy-1,8-naphthalenedicarboximide and N,N'-bis(methanesulfonyloxy)-1,8;4,5-naphthalenetetracarboxylic diimide; and compounds of Formula (3) where X is an -OR group and R is a group corresponding to a moiety of an inorganic acid other than hydroxyl group, such as sulfuric esters, nitric esters, phosphoric esters, and boric esters of N-hydroxy-1,8-naphthalenedicarboximide and N,N'-dihydroxy-1,8;4,5-naphthalenetetracarboxylic diimide.

In addition to the cyclic imide compounds, the nitrogen-containing cyclic compounds each having a skeleton of Formula (I) as a constituent of its ring further include cyclic acylurea compounds having a cyclic acylurea skeleton [-C(=O)-N-C(=O)-N-]. Representative examples of such cyclic acylurea compounds include hydro-1-hydroxy (or 1-substituted oxy)-1,3,5-triazine-2,6-dione compounds of following Formula (6): 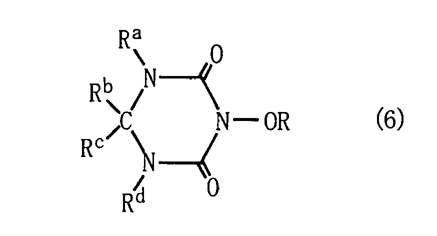 wherein R^{a} and R^{d} are each independently one of hydrogen atom, an alkyl group, an aryl group, a cycloalkyl group, a protected or unprotected hydroxyl group, a protected or unprotected carboxyl group, and an acyl group; R^{b} and R^{c} are each independently one of hydrogen atom, a halogen atom, an alkyl group, an aryl group, a cycloalkyl group, hydroxyl group, an alkoxy group, carboxyl group, a substituted oxycarbonyl group, an acyl group, and an acyloxy group, in which at least two of R^{a}, R^{b}, R^{c} and R^{d} may be combined to form a double bond, or an aromatic or non-aromatic ring together with an atom constituting the ring in Formula (6), and R^{b} and R^{c} may together form oxo group; and R is as defined above.

In Formula (6), examples of the alkyl groups, aryl groups, cycloalkyl groups, and acyl groups as R^{a} and R^{d} are as with the corresponding groups exemplified in connection with R¹ to R⁶, and examples of the hydroxyl-protecting groups are as with above.

The carboxyl-protecting groups may be protecting groups commonly used in organic synthesis, and examples thereof include alkoxy groups including alkoxy groups having about one to about six carbon atoms, such as methoxy, ethoxy, and butoxy; cycloalkyloxy groups; aryloxy groups such as phenoxy group; aralkyloxy groups such as benzyloxy group; trialkylsilyloxy groups such as trimethylsilyloxy group; substituted or unsubstituted amino groups including amino group, and mono- or di-alkyl-amino groups whose alkyl moieties each have about one to about six carbon atoms, such as methylamino group and dimethylamino group.

Examples of the halogen atoms, alkyl groups, aryl groups, cycloalkyl groups, hydroxyl group, alkoxy groups, carboxyl group, substituted oxycarbonyl groups, acyl groups, and acyloxy groups as R^{b} and R^{c} are as with the corresponding groups exemplified in connection with R¹ to R⁶.

In Formula (6), at least two of R^{a}, R^{b}, R^{c}, and R^{d} may be combined to form a double bond, or an aromatic or non-aromatic ring with at least one atom (carbon atom and/or nitrogen atom) constituting the ring, and R^{b} and R^{c} may together form oxo group. Preferred examples of the aromatic or non-aromatic ring are as above.

Among compounds of Formula (6), preferred are isocyanuric acid derivatives of following Formula (6a): 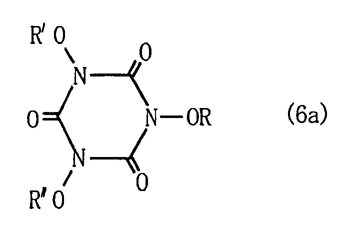 wherein each of R, R', and R" is independently hydrogen atom or a hydroxyl-protecting group.

Representative examples of the cyclic acylurea compounds include hexahydro-1,3,5-trihydroxy-1,3,5-triazine-2,4,6-trione (i.e., 1,3,5-trihydroxyisocyanuric acid), 1,3,5-triacetoxy-hexahydro-1,3,5-triazine-2,4,6-trione, hexahydro-1,3,5-tris(methoxymethyloxy)-1,3,5-triazine-2,4,6-trione, hexahydro-1-hydroxy-1,3,5-triazine-2,6-dione, hexahydro-1-hydroxy-3,5-dimethyl-1,3,5-triazine-2,6-dione, 1-acetoxy-hexahydro-1,3,5-triazine-2,6-dione, and 1-acetoxy-hexahydro-3,5-dimethyl-1,3,5-triazine-2,6-dione.

Among the nitrogen-containing cyclic compounds, compounds where X is an -OR group and R is hydrogen atom (N-hydroxy cyclic compounds) can be prepared according to a known procedure or according to a combination of known procedures. Among the nitrogen-containing cyclic compounds, compounds where X is an -OR group and R is a hydroxyl-protecting group can each be prepared by introducing a desired protecting group into a corresponding compound where R is hydrogen atom (N-hydroxy cyclic compound) using a common reaction for the introduction of protecting groups.

More specifically, among the cyclic imide compounds, compounds where X is an -OR group and R is hydrogen atom can be prepared through a common imidization process (a process for the formation of an imide), such as a process that includes the steps of reacting a corresponding acid anhydride with hydroxylamine for ring-opening of an acid anhydride group, and closing the ring to form an imide. N-acetoxyphthalimide can be prepared, for example, by reacting N-hydroxyphthalimide with acetic anhydride or by reacting N-hydroxyphthalimide with an acetyl halide in the presence of base. The compounds can also be prepared by other processes.

Typically preferred cyclic imide compounds as catalysts include N-hydroxy imide compounds (e.g., N-hydroxysuccinimide, N-hydroxyphthalimide, N,N'-dihydroxypyromellitic diimide, N-hydroxyglutarimide, N-hydroxy-1,8-naphthalenedicarboximide, and N,N'-dihydroxy-1,8:4,5-naphthalenetetracarboxylic diimide) derived from aliphatic polycarboxylic acid anhydrides (cyclic anhydrides) or aromatic polycarboxylic acid anhydrides (cyclic anhydrides); and compounds corresponding to these N-hydroxy imide compounds, except for introducing protecting group into hydroxyl group thereof.

Among the cyclic acylurea compounds, 1,3,5-triacetoxy-hexahydro-1,3,5-triazine-2,4,6-trione (i.e., 1,3,5-triacetoxyisocyanuric acid), for example, can be prepared by reacting hexahydro-1,3,5-trihydroxy-1,3,5-triazine-2,4,6-trione (i.e., 1,3,5-trihydroxyisocyanuric acid) with acetic anhydride or by reacting hexahydro-1,3,5-trihydroxy-1,3,5-triazine-2,4,6-trione with an acetyl halide in the presence of base.

Each of the nitrogen-containing cyclic compounds having a skeleton of Formula (I) as a constituent of its ring can be used alone or in combination. The nitrogen-containing cyclic compound(s) may be formed within the reaction system. The nitrogen-containing cyclic compound(s) may be used as supported by a support. The support for use herein is often a porous support (carrier) such as activated carbon, zeolite, silica, silica-alumina, or bentonite. The amount of the nitrogen-containing cyclic compound(s) relative to the support is, for example, about 0.1 to about 50 parts by weight, preferably about 0.5 to about 30 parts by weight, and more preferably about 1 to about 20 parts by weight, to 100 parts by weight of the support.

The amount of the nitrogen-containing cyclic compound(s) can be set within broad ranges and is, for example, about 0.0000001 to about 1 mole, preferably about 0.0001 to about 0.5 mole, more preferably about 0.001 to about 0.4 mole, and particularly preferably about 0.01 to about 0.35 mole, to 1 mole of the substituted diarylethane compound of Formula (1).

One or more promoters (co-catalysts) can be used in combination with the nitrogen-containing cyclic compound(s). Examples of the promoters include metallic compounds. By using a metallic compound as a promoter, the rate and/or selectivity of the reaction can be improved. Among metallic compounds, transition metal compounds are preferred. Examples of transition metal compounds are halides, organic acid salts, oxo acid salts, and complexes of transition metals [elements belonging to Groups 3 to 12 of the Periodic Table of Elements, such as V, Mo, Mn, Fe, Ru, Co, and Cu]. Taking cobalt compounds as an example, more specific examples are cobalt chloride, cobalt bromide, cobalt acetate, cobalt octylate, cobalt naphthenate, cobalt sulfate, cobalt nitrate, and cobalt acetylacetonate. Cobalt compounds and manganese compounds are preferred as the transition metal compounds. The amount of the metallic compound(s) is, for example, about 0.001 to about 0.1 mole, and preferably about 0.005 to about 0.08 mole, to 1 mole of the nitrogen-containing cyclic compound(s).

In an embodiment of the present invention, there may be used, as a promoter, one or more organic salts each composed of a polyatomic cation or a polyatomic anion and its counter ion, which polyatomic cation or anion contains an element belonging to Group 15 or Group 16 of the Periodic Table of Elements having at least one organic group bonded thereto. Use of the organic salt(s) as a promoter can improve the rate and/or selectivity of the reaction.

The reaction system herein may further include a free-radical generator or a free-radical reaction accelerator. Examples of such components include halogens such as chlorine and bromine; peracids such as peracetic acid and m-chloroperbenzoic acid; peroxides including hydroperoxides such as hydrogen peroxide and t-butyl hydroperoxide (TBHP); and azo compounds such as azobisisobutyronitrile. The existence of these components in the system may enhance the oxidation reaction. The amount of the above-mentioned component is, for example, about 0.001 to about 2 moles, preferably about 0.01 to about 1 mole, and more preferably about 0.05 to about 0.8 mole, to 1 mole of the nitrogen-containing cyclic compound.

### [Reaction]

In an embodiment of the present invention, a substituted diarylethane compound of Formula (1) is oxidized with oxygen in the presence of a nitrogen-containing cyclic compound containing a skeleton of Formula (I) as a constituent of its ring, and the oxidized product is treated with acid.

The oxidation reaction of the substituted diarylethane compound of Formula (1) is generally conducted in an organic solvent. Examples of such organic solvents include nitriles such as acetonitrile, propionitrile, and benzonitrile; amides such as N,N-dimethylformamide; halogenated hydrocarbons such as chlorobenzene and trifluoromethylbenzene; nitro compounds such as nitrobenzene and nitromethane; esters such as ethyl acetate and butyl acetate; organic acids such as formic acid, acetic acid, propionic acid, butyric acid, and isobutyric acid, of which aliphatic saturated carboxylic acids are preferred; and mixtures of these solvents. As the solvent, often used are nitriles such as acetonitrile and benzonitrile; and esters such as ethyl acetate.

Molecular oxygen can be used as the oxygen. The molecular oxygen is not particularly limited and can be, for example, any of pure oxygen; diluted oxygen with an inert gas such as nitrogen, helium, argon, or carbon dioxide; air; or diluted air. The oxygen may be formed within the reaction system. The amount of oxygen can be appropriately set depending typically on the type of the substrate, substituted diarylethane compound, and is generally about 0.5 mole or more, for example, about 1 mole or more, preferably about 1 to about 100 moles, and more preferably about 2 to about 50 moles, to 1 mole of the substituted diarylethane compound. Oxygen is often used in excess moles to the substituted diarylethane compound.

The reaction temperature of the oxidation reaction can be set appropriately according typically to the type of the substituted diarylethane compound and is, for example, about 30°C to about 150°C, preferably about 40°C to about 120°C, and more preferably about 50°C to about 100°C. The reaction can be conducted under normal pressure or under a pressure (under a load). The reaction pressure is generally about 0.1 to about 10 MPa, and preferably about 0.1 to about 5 MPa. The reaction time (duration) may be set according to the reaction temperature and reaction pressure within ranges of, for example, about 10 minutes to about 48 hours, and preferably about 1 to about 24 hours. The reaction can be conducted in the presence of or under flow of oxygen according to a common system such as batch system, semi-batch system, or continuous system.

As a result of the oxidation reaction, a tertiary carbon atom in the substituted diarylethane compound of Formula (1) is probably oxidized to yield a corresponding hydroperoxide. The tertiary carbon atom is a carbon atom to which two aryl groups (groups relating to Ring Ar¹ and Ring Ar², respectively) and a methyl group are bound.

After the completion of oxidation reaction, a treatment with acid is carried out, for example, by adding an acid to the reaction mixture. Examples of the acid include inorganic acids such as sulfuric acid, hydrochloric acid, nitric acid, and phosphoric acid; sulfonic acids such as methanesulfonic acid, trifluoromethanesulfonic acid, and p-toluenesulfonic acid; strongly acidic cation-exchange resins; and sulfur dioxide and sulfur trioxide. The acid may be formed into a diluted solution, such as an aqueous solution, before use. The acid treatment may be carried out at a temperature of, for example, about -40°C to about 40°C, preferably about -20°C to about 25°C, and more preferably about -10°C to about 10°C.

The acid treatment selectively yields a target compound, phenolic compound of Formula (2). This treatment simultaneously yields an arylmethyl ketone, such as an acetophenone compound, of following Formula (7): 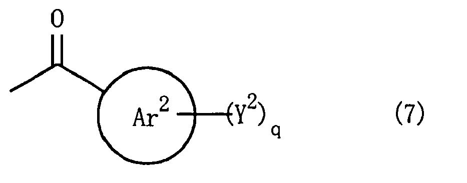 wherein Ring Ar², Y², q are as defined above. Specifically, when the hydroperoxide formed by oxidation is decomposed with an acid, one aryl group (of Ring Ar¹) to which an electron-donating group is bound is selectively converted into a phenolic compound, and the other aryl group (of Ring Ar²) is selectively converted into an arylmethyl ketone. According to an embodiment of the present invention, therefore, phenolic compounds each having an electron-donating group such as an alkyl group or an alkoxy group can be industrially efficiently prepared.

The reaction product after the completion of reaction can be separated and purified, for example, by a separation procedure such as filtration, concentration, distillation, extraction, crystallization, recrystallization, adsorption, or column chromatography, or any combination of these.

The substituted phenolic compounds can be used typically as materials for phenolic resins, synthetic dyestuffs, plasticizers, medicaments, and agricultural chemicals.

### Examples

The present invention will be illustrated in further detail with reference to several examples below, which, however, are by no means construed to limit the scope of the present invention.

### Preparation Example 1

To o-xylene (200 mL) were added styrene (20 mmol) and FeCl₃.6H₂O (2 mmol), followed by stirring at 80°C for 4 hours. After the reaction, the reaction mixture was washed with water to remove the catalyst, purified by distillation, and thereby yielded 1,2-dimethyl-4-(1-phenylethyl)benzene in a yield of 80%.

### Preparation Example 2

To o-xylene (200 mL) were added 4-chlorostyrene (20 mmol) and FeCl₃.6H₂O (2 mmol), followed by stirring at 80°C for 4 hours. After the reaction, the reaction mixture was washed with water to remove the catalyst, purified by distillation, and thereby yielded 4-[1-(4-chlorophenyl)ethyl]-1,2-dimethylbenzene in a yield of 89%.

### Preparation Example 3

To anisole (200 mL) were added styrene (20 mmol) and FeCl₃.6H₂O (2 mmol), followed by stirring at 80°C for 4 hours. After the reaction, the reaction mixture was washed with water to remove the catalyst, purified by distillation, and thereby yielded 4-(1-phenylethyl)anisole in a yield of 80%.

### Preparation Example 4

To 1,2-dimethoxybenzene (200 mL) were added styrene (20 mmol) and FeCl₃.6H₂O (2 mmol), followed by stirring at 80°C for 4 hours. After the reaction, the reaction mixture was washed with water to remove the catalyst, purified by distillation, and thereby yielded 1,2-dimethoxy-4-(1-phenylethyl)benzene in a yield of 59%.

### Preparation Example 5

To 1,4-dimethoxybenzene (200 mL) were added styrene (20 mmol) and FeCl₃.6H₂O (2 mmol), followed by stirring at 80°C for 4 hours. After the reaction, the reaction mixture was washed with water to remove the catalyst, purified by distillation, and thereby yielded 1,4-dimethoxy-3-(1-phenylethyl)benzene in a yield of 51%.

### Example 1

In a reactor were placed 1,2-dimethyl-4-(1-phenylethyl)benzene (10 mmol), N-hydroxyphthalimide (1 mmol), azobisisobutyronitrile (0.3 mmol), and acetonitrile (3 mL), followed by stirring at 75°C in an atmosphere of oxygen gas under normal pressure for 6 hours. The reaction mixture was cooled to 0°C, combined with 0.1-M H₂SO₄ (10 mL) added dropwise, and stirred for 30 minutes. The reaction mixture was then neutralized with an aqueous ammonia solution and analyzed by gas chromatography to find that there were produced 3,4-dimethylphenol in a yield of 46%, acetophenone in a yield of 50%, 3,4-dimethylacetophenone in a yield of 2.7%, and phenol in a trace amount, with a conversion from 1,2-dimethyl-4-(1-phenylethyl)benzene of 64%.

### Example 2

In a reactor were placed 1,2-dimethyl-4-(1-phenylethyl)benzene (10 mmol), N-hydroxyphthalimide (1 mmol), azobisisobutyronitrile (0.3 mmol), and acetonitrile (3 mL), followed by stirring at 75°C in an atmosphere of oxygen gas under normal pressure for 6 hours. The reaction mixture was then cooled to 0°C, combined with 0.5 g of a cation-exchange resin (trade name: "AMBERLYST 15J"), and stirred for 1 hour. The reaction mixture was analyzed by gas chromatography to find that there were produced 3,4-dimethylphenol in a yield of 50%, acetophenone in a yield of 50%, 3,4-dimethylacetophenone in a yield of 1%, and phenol in a trace amount, with a conversion from 1,2-dimethyl-4-(1-phenylethyl)benzene of 62%.

### Example 3

The procedure of Example 1 was repeated, except for using 4-[1-(4-chlorophenyl)ethyl]-1,2-dimethylbenzene instead of 1,2-dimethyl-4-(1-phenylethyl)benzene. The reaction mixture was analyzed by gas chromatography to find that there were produced 3,4-dimethylphenol in a yield of 43%, 4-chloroacetophenone in a yield of 48%, and 3,4-dimethylacetophenone in a yield of 2.4%, with a conversion from 4-[1-(4-chlorophenyl)ethyl]-1,2-dimethylbenzene of 63%, and that 4-chlorophenol was not detected.

### Example 4

The procedure of Example 1 was repeated, except for using 4-(1-phenylethyl)anisole instead of 1,2-dimethyl-4-(1-phenylethyl)benzene. The reaction mixture was analyzed by gas chromatography to find that there were produced 4-methoxyphenol in a yield of 54% and acetophenone in a yield of 54% with a conversion from 4-(1-phenylethyl)anisole of 54%, and that phenol was not detected.

### Example 5

The procedure of Example 1 was repeated, except for using 1,2-dimethoxy-4-(1-phenylethyl)benzene instead of 1,2-dimethyl-4-(1-phenylethyl)benzene. The reaction mixture was analyzed by gas chromatography to find that there were produced 3,4-dimethoxyphenol in a yield of 48% and acetophenone in a yield of 55%, with a conversion from 1,2-dimethoxy-4-(1-phenylethyl)benzene of 64%, and that phenol was not detected.

### Example 6

The procedure of Example 1 was repeated, except for using 1,4-dimethoxy-3-(1-phenylethyl)benzene instead of 1,2-dimethyl-4-(1-phenylethyl)benzene. The reaction mixture was analyzed by gas chromatography to find that there were produced 2,5-dimethoxyphenol in a yield of 22%, acetophenone in a yield of 25%, and phenol in a trace amount, with a conversion from 1,4-dimethoxy-3-(1-phenylethyl)benzene of 36%.

It should be understood by those skilled in the art that various modifications, combinations, subcombinations, and alterations may occur depending on design requirements and other factors insofar as they are within the scope of the appended claims or the equivalents thereof.

## Claims

1. A process for the preparation of a substituted phenolic compound, the process comprising the steps of:
oxidizing a substituted diarylethane compound with oxygen in the presence of a nitrogen-containing cyclic compound to give an oxidized product; and
treating the oxidized product with an acid to give the substituted phenolic compound,
wherein the nitrogen-containing cyclic compound includes, as a constituent of its ring, a skeleton represented by following Formula (I): 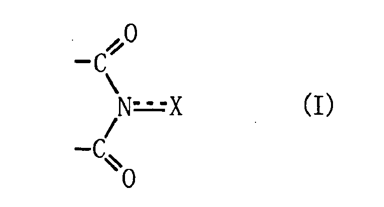 wherein X represents oxygen atom or an -OR group, where R represents hydrogen atom or a hydroxyl-protecting group,
the substituted diarylethane compound is represented by following Formula (1): 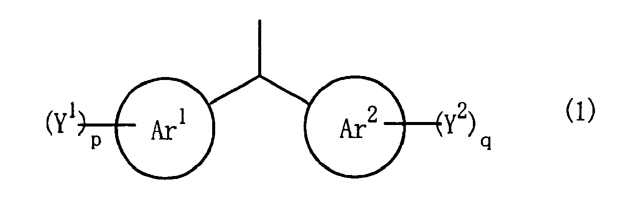 wherein Ring Ar¹ and Ring Ar² each independently represent a monocyclic or polycyclic aromatic carbocyclic ring;
Y¹ represents an electron-donating group bound to Ring Ar¹;
Y² represents an electron-withdrawing group bound to Ring Ar²;
"p" denotes an integer of 1 or more; and
"q" denotes an integer of 0 or more,
wherein, when "p" is an integer of 2 or more and there are two or more Y¹s, the two or more Y¹s may be the same as or different from one another and they may be combined to form a ring with carbon atom on Ring Ar¹, and
wherein, when "q" is an integer of 2 or more and there are two or more Y²s, the two or more Y²s may be the same as or different from one another and they may be combined to form a ring with carbon atom on Ring Ar², and
the substituted phenolic compound is represented by following Formula (2): 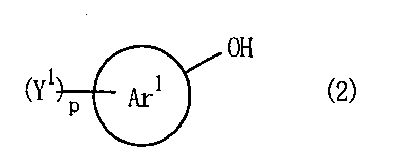 wherein Ring Ar¹, Y¹, and "p" are as defined above.

2. The process according to claim 1, wherein the nitrogen-containing cyclic compound is a compound represented by following Formula (3): 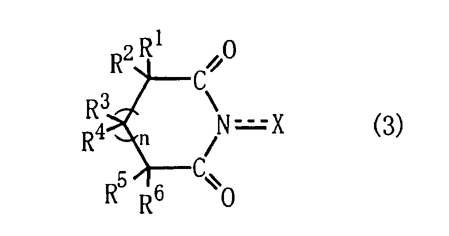 wherein "n" is 0 or 1;
X represents oxygen atom or an -OR group, where R represents hydrogen atom or a hydroxyl-protecting group; and
R¹, R², R³, R⁴, R⁵, and R⁶ each independently represent one selected from the group consisting of hydrogen atom, a halogen atom, an alkyl group, an aryl group, a cycloalkyl group, hydroxyl group, an alkoxy group, carboxyl group, a substituted oxycarbonyl group, an acyl group, and an acyloxy group,
wherein at least two of R¹, R², R³, R⁴, R⁵, and R⁶ may be combined to form a double bond, or an aromatic or non-aromatic ring together with carbon atom or carbon-carbon bond constituting the cyclic imide skeleton, and
wherein one or more N-substituted cyclic imido groups may be further formed on at least one of R¹, R², R³, R⁴, R⁵, and R⁶, and/or on at least one of the double bond and the aromatic or non-aromatic ring formed by at least two of R¹, R², R³, R⁴, R⁵, and R⁶, wherein the N-substituted cyclic imido groups are each represented by following Formula (a): 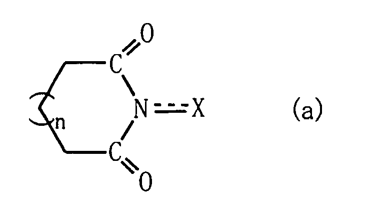 wherein "n" and X are as defined above.

3. The process according to claim 1, wherein the electron-donating group as Y¹ is a substituent selected from the group consisting of an alkyl group, a cycloalkyl group, hydroxyl group, mercapto group, a substituted oxy group, a substituted thio group, amino group, and a mono- or di-substituted amino group.
